# EUROPÄISCHE PATENTSCHRIFT

(11) **EP 2 047 271 B1**
(45) Veröffentlichungstag und Bekanntmachung des Hinweises auf die Patenterteilung: **14.11.2012**
(21) Anmeldenummer: 07817416.6
(22) Anmeldetag: 01.08.2007
(51) Int. Cl.: G01N 33/574, A61P 35/00

(54) **VERFAHREN ZUR FESTSTELLUNG DER SENSITIVITÄT VON TUMOREN GEGENÜBER CAPECITABIN**
METHOD FOR ESTABLISHING THE SENSITIVITY OF TUMOURS TO CAPECITABIN
PROCÉDÉ DE DÉTERMINATION DE LA SENSIBILITÉ DE TUMEURS À LA CAPÉCITABINE

(30) Priorität: 02.08.2006 DE 102006037158
(43) Veröffentlichungstag der Anmeldung: 15.04.2009
(73) Patentinhaber: Sanoxsys GmbH, 80799 München (DE)
(72) Erfinder: SCHALLER, Gerhard, 80639 München (DE)
(74) Vertreter: Baumbach, Friedrich
(86) Internationale Anmeldenummer: PCT/DE2007/001369
(87) Internationale Veröffentlichungsnummer: WO 2008/014779

(56) Entgegenhaltungen:
- EP-A1- 1 510 588
- EP-B1- 1 140 192
- WO-A2-2007/103823
- MIWA M ET AL: "Design of a novel oral fluoropyrimidine carbamate, capecitabine, which generates 5-fluorouracil selectively in tumours by enzymes concentrated in human liver and cancer tissue." EUROPEAN JOURNAL OF CANCER (OXFORD, ENGLAND : 1990) JUL 1998, Bd. 34, Nr. 8, Juli 1998 (1998-07), Seiten 1274-1281, XP002459166 ISSN: 0959-8049
- ENDO M ET AL: "Induction of thymidine phosphorylase expression and enhancement of efficacy of capecitabine or 5'-deoxy-5-fluorouridine by cyclophosphamide in mammary tumor models." INTERNATIONAL JOURNAL OF CANCER. JOURNAL INTERNATIONAL DU CANCER 24 SEP 1999, Bd. 83, Nr. 1, 24. September 1999 (1999-09-24), Seiten 127-134, XP002459167 ISSN: 0020-7136
- SAWADA N ET AL: "Induction of thymidine phosphorylase activity and enhancement of capecitabine efficacy by taxol/taxotere in human cancer xenografts." CLINICAL CANCER RESEARCH : AN OFFICIAL JOURNAL OF THE AMERICAN ASSOCIATION FOR CANCER RESEARCH APR 1998, Bd. 4, Nr. 4, April 1998 (1998-04), Seiten 1013-1019, XP002459168 ISSN: 1078-0432
- MILLER KATHY D ET AL: "Randomized phase III trial of capecitabine compared with bevacizumab plus capecitabine in patients with previously treated metastatic breast cancer." JOURNAL OF CLINICAL ONCOLOGY : OFFICIAL JOURNAL OF THE AMERICAN SOCIETY OF CLINICAL ONCOLOGY 1 FEB 2005, Bd. 23, Nr. 4, 1. Februar 2005 (2005-02-01), Seiten 792-799, XP002459169 ISSN: 0732-183X
- BLANQUICETT CARMELO ET AL: "Antitumor efficacy of capecitabine and celecoxib in irradiated and lead-shielded, contralateral human BxPC-3 pancreatic cancer xenografts: clinical implications of abscopal effects." CLINICAL CANCER RESEARCH : AN OFFICIAL JOURNAL OF THE AMERICAN ASSOCIATION FOR CANCER RESEARCH 15 DEC 2005, Bd. 11, Nr. 24 Pt 1, 15. Dezember 2005 (2005-12-15), Seiten 8773-8781, XP002459170 ISSN: 1078-0432
- PENTHEROUDAKIS G ET AL: "THE RATIONAL DEVELOPMENT OF CAPECITABINE FROM THE LABORATORY TO THE CLINIC" ANTICANCER RESEARCH, HELENIC ANTICANCER INSTITUTE, ATHENS,, GR, Bd. 22, Nr. 6B, 2002, Seiten 3589-3596, XP009048135 ISSN: 0250-7005
- SAIF M WASIF ET AL: "Phase I study of capecitabine with concomitant radiotherapy for patients with locally advanced pancreatic cancer: expression analysis of genes related to outcome." JOURNAL OF CLINICAL ONCOLOGY : OFFICIAL JOURNAL OF THE AMERICAN SOCIETY OF CLINICAL ONCOLOGY 1 DEC 2005, Bd. 23, Nr. 34, 1. Dezember 2005 (2005-12-01), Seiten 8679-8687, XP002459171 ISSN: 0732-183X
- AKIYAMA SHIN-ICHI ET AL: "The role of thymidine phosphorylase, an angiogenic enzyme, in tumor progression." CANCER SCIENCE NOV 2004, Bd. 95, Nr. 11, November 2004 (2004-11), Seiten 851-857, XP002459172 ISSN: 1347-9032
- YAMASHITA HIDEKI ET AL: "Cyclooxygenase-2 in human malignant fibrous histiocytoma: correlations with intratumoral microvessel density, expression of vascular endothelial growth factor and thymidine phosphorylase." INTERNATIONAL JOURNAL OF MOLECULAR MEDICINE OCT 2004, Bd. 14, Nr. 4, Oktober 2004 (2004-10), Seiten 565-570, XP008086019 ISSN: 1107-3756
- ACKLAND S P ET AL: "Thymidine phosphorylase: Its role in sensitivity and resistance to anticancer drugs", DRUG RESISTANCE UPDATES 199908 GB LNKD- DOI:10.1054/DRUP.1999.0089, vol. 2, no. 4, August 1999 (1999-08), pages 205-214, ISSN: 1368-7646
- ISHIKAWA T ET AL: "POSITIVE CORRELATION BETWEEN THE EFFICACY OF CAPECITABINE AND DOXIFLURIDINE AND THE RATIO OF THYMIDINE PHOSPHORYLASE TO DIHYDROPYRIMIDINE DEHYDROGENASE ACTIVITIES IN TUMORS IN HUMAN CANCER XENOGRAFTS", CANCER RESEARCH, AMERICAN ASSOCIATION FOR CANCER REREARCH, US, vol. 58, no. 4, 15 February 1998 (1998-02-15), pages 685-690, XP008025976, ISSN: 0008-5472
- ZHU ANDREW X ET AL: "Chemotherapeutic and biologic agents as radiosensitizers in rectal cancer.", SEMINARS IN RADIATION ONCOLOGY OCT 2003 LNKD- PUBMED:14586834, vol. 13, no. 4, October 2003 (2003-10), pages 454-468, ISSN: 1053-4296
- JOHNSON M L ET AL: "Emerging targeted therapies for breast cancer", ONCOLOGY 2005 US, vol. 19, no. 5, 2005, pages 611-618, XP008129927, ISSN: 0890-9091
- PARK D J ET AL: "Thymidylate synthase gene polymorphism predicts response to capecitabine in advanced colorectal cancer", COMPUTATIONAL AND THEORETICAL POLYMER SCIENCE, PRA PRESS, CINCINNATI, OH, US, vol. 17, no. 1, 1 January 2002 (2002-01-01), pages 46-49, XP002347623, ISSN: 1089-3156
- LEICHMAN C G ET AL: "QUANTITATION OF INTRATUMORAL THYMIDYLATE SYNTHASE EXPRESSION PREDICTS FOR DISSEMINATED COLORECTAL CANCER RESPONSE AND RESISTANCE TO PROTRACTED-INFUSION FLUOROURACIL AND WEEKLY LEUCOVORIN", JOURNAL OF CLINICAL ONCOLOGY, AMERICAN SOCIETY OF CLINICAL ONCOLOGY, US, vol. 15, no. 10, 1 October 1997 (1997-10-01), pages 3223-3229, XP001013038, ISSN: 0732-183X
- AI-WEN WU ET AL: 'COX-2 expression and tumor angiogenesis in colorectal cancer' WORLD J GASTROENTEROL 2004;10(16), [Online] 01 Januar 2004, Seiten 2323 - 2326, XP055012993 Gefunden im Internet: <URL:http://www.wjgnet.com/1007-9327/10/232 3.pdf> [gefunden am 2011-11-24]
- HUALI JIN ET AL: 'Protein/DNA vaccine-induced antigen-specific Treg confer protection against asthma' EUROPEAN JOURNAL OF IMMUNOLOGY Bd. 38, Nr. 9, 01 September 2008, Seiten 2451 - 2463, XP055013095 ISSN: 0014-2980

## Beschreibung

Die Erfindung betrifft ein Verfahren zur Identifizierung eines humanen Tumors wie beansprucht.

Anwendungsgebiete der Erfindung sind die pharmazeutische Industrie und die Lebenswissenschaften: die Biologie, die Biochemie, die Biotechnologie, die Medizin und die Medizintechnologie.

Die Behandlung metastasierter Tumoren ist eine enorme Herausforderung für Onkologen. Beispielsweise entwickeln 30-40% aller Patientinnen mit Brustkrebs in westlichen Ländern ein metastasierendes Mammakarzinom.

Capecitabin (IUPAC-Name: Pentyl[1-(3,4-dihydroxy-5-methyl-tetrahydrofuran-2-yl)-5-fluoro-2-oxo-1H-pyrimidin-4-yl]aminomethanoat) ist ein Chemotherapeutikum, das regelmäßig in der Behandlung von metastasierten Tumoren der Brust und des Darms oder anderer solider Tumoren verwendet wird.

Capecitabin wird im Körper, zuletzt vorzugsweise im Tumor, zu der Verbindung 5-Fluoruracil (5-FU) umgewandelt. 5-FU wird bei der Zellteilung aufgrund seiner Strukturähnlichkeit mit Cytosin und Thymin statt dieser in die DNA eingebaut. Die dabei gebildete DNA ist nicht funktionsfähig. PersonalisierteTherapien werden in Johnson & Seidman, Oncology 2005, offenbart.

### Fluoropyrimidine in der Therapie des metastasierten Mammakarzinoms

Hansen berichtete 1987 erstmals über den Einsatz von einer Therapie mit kontinuierlicher Gabe von low dose 5-FU bei intensivst vorbehandelten Patientinnen mit fortgeschrittenem Mammakarzinom (Hansen et al., 1987). Von 25 Patienten erreichten 8 ein objektives Ansprechen. Toxizitäten wie Hand-Fuß-Syndrom, Mukositis, Diarrhoen konnten durch kurzfristige Therapieunterbrechung oder Dosisreduktion behoben werden. Diese Ergebnisse konnten durch Jabboury 1989, Huan 1989 und durch Hansen 1991 wiederholt bestätigt werden.
Dauerinfusionen sind jedoch technisch aufwändig, teuer, arbeitsintensiv und vor allem für Patienten unbequem und zusätzlich durch Komplikationen des zentralvenösen Katheters belastet. Die orale Gabe eines Fluoropyrimidins stellt demgegenüber eine elegante Methode dar, den Wirkmechanismus der protrahierten oder 5-FU Dauerinfusion zu imitieren.

Capecitabin ist ein orales Fluorpyrimidincarbamat. Capecitabin selbst wirkt nicht zytotoxisch, wird jedoch durch drei enzymatische Schritte hauptsächlich im Tumor zum aktiven zytotoxischen Metaboliten Fluoruracil (5-FU) umgewandelt. Fluoruracil gehört zur Gruppe der Antimetaboliten, als Pyrimidinantagonist konkurriert es mit dem eigentlichen Substrat und führt zur Hemmung der Nukleinsäuresynthese. Der Einbau von 5-FU anstelle von Uracil führt zu einer Inhibierung der RNA und Proteinsynthese. Durch Blockade der Methylierung von Desoxyuridylsäure kommt es zur Bildung von Thymidylsäure, wodurch zusätzlich die Synthese der DNA beeinflusst wird. Am stärksten treffen die Auswirkung der DNA- und RNA-Deprivation jene Zellen, die schneller proliferieren und Capecitabin schneller zu 5-FU metabolisieren.

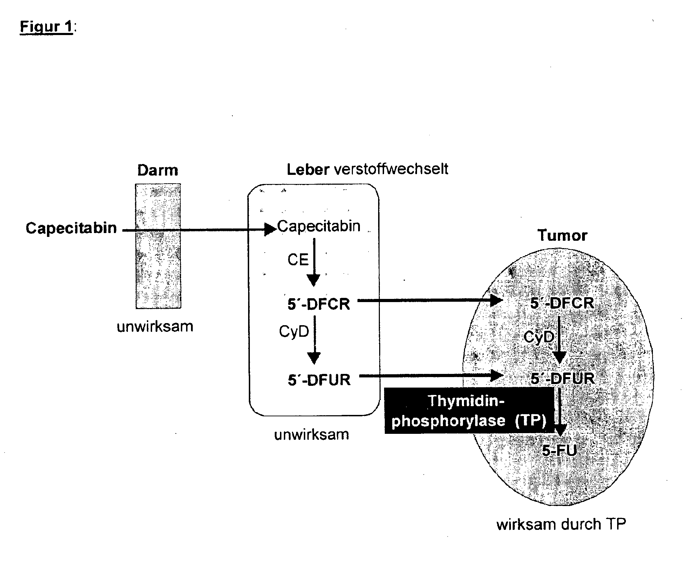

Nach oraler Aufnahme wird Capecitabin zunächst durch hepatische Carboxylesterasen zu 5'-Desoxy-5-Fluorcytidin (5'DFCR) metabolisiert, das wiederum durch die Cytidin-Desaminase, die sich hauptsächlich in der Leber und im Tumorgewebe befindet, in 5'-Desoxy-5-Fluoruridin (5'-DFUR) umgewandelt wird (Figur 1). In der Folge wird 5'DFUR durch die Thymidinphosphorylase (TP) vornehmlich im Tumor katalytisch aktiviert (Ishitsuka et al., 1995). Die Thymidinphosphorylase, auch unter dem Namen "Tumor Assoziierter Angiogenese Faktor", liegt in hoher Konzentration vorwiegend in malignen Zellen vor. Entsprechend ihrer Expression im Tumor lässt sich eine tumorale und eine peritumorale Fraktion unterscheiden. Die peritumorale Fraktion induziert bei den gesunden Zellen, die den Tumor umgeben die Bildung von VEGF (Vascular Endothelial Growth Factor), (Nicholas S. Brown et al.: Thymidine Phosphorylase Induces Carcinoma Cell Oxidative Stress and Promotes Secretion of Angiogenic Factors. Cancer Research 60 (2000) 6298-6302) was wiederum zu einer verstärkten Gefäßneubildung im Bereich des Tumor führt. Dementsprechend korreliert die TP mit schnellem Malignomwachstum, aggressivem Invasionsverhalten und schlechter Prognose (Folkman et al., 1996). Daher sollten gerade solche Patienten mit schlechter Prognose von einer Capecitabintherapie profitieren.

Capecitabin wirkt additiv mit anderen zytotoxischen Substanzen. In vitro Untersuchungen konnten additive Effekte mit CPT-11, Tomudex , Mitomycin C oder Cyclophosphamid und supra-additive mit Paclitaxel oder Docetaxel zeigen. Synergistische Effekte bestehen zwischen Capecitabin und einer Strahlentherapie. Diese Effekte wurden nicht unter 5-FU beobachtet und sind zurückzuführen auf die Hochregulation des Schlüsselenzyms beim Capecitabinmetabolismus durch die genannten Zytostatika aber auch durch die Bestrahlung (Sawada et al., 1998 ,1999).

Das Medikament Capecitabin (Markenname: Xeloda®) wurde 2001 für die Therapie des metastasierten Dickdarmkarzinoms zugelassen. Im Jahr darauf erfolgte die Zulassung des Medikaments in Kombination mit der Gabe von Docetaxel (Taxotere) für die Behandlung des metastasierten Mammakarzinoms. Dem zugrunde lag eine klinische Phase III-Studie bei der Capecitabin in Kombination mit Docetaxel (Taxotere) gegen Docetaxel allein geprüft wurde. Erstmalig konnte in dieser Studie eine signifikante Lebensverlängerung für Patientinnen mit einem metastasierten Brustkrebs erreicht werden. Obwohl die Wirkung von Capecitabin als sog. Pro-drug-Chemotherapeutikum zwingend an das Vorhandensein des Enzyms Thymidinphosphorylase gebunden ist, erfolgte die Zulassung ohne einen vorgeschalteten Nachweis. Dies gilt auch für alle anderen Indikationsfelder von Capecitabin.

### Ergebnisse klinischer Studien

Capecitabin wurde beim metastasierten Mammakarzinom als Erst-, Zweit- und Drittlinientherapie in klinischen Studien geprüft.

In einer Phase II Studie (Blum et al., 1998) erhielten 162 massiv vorbehandelte Patientinnen mit metastasiertem Mammakarzinom Capecitabin in einer Dosierung von 2510mg/m²/Tag über 14 Tage, gefolgt von einer 7-tägigen Therapiepause. Die Behandlung wurde in 3-Wochen-Zyklen weitergeführt.

Die Dauer der Therapie richtete sich nach dem Verlauf. Patientinnen, die auf die Therapie mit Capecitabin ansprachen, wurden bis zum Tumorprogress weiterbehandelt.
Als Einschlusskriterium galt eine primäre oder sekundäre Resistenz oder ein Therapieversagen auf eine Chemotherapie mit Paclitaxel, 90% hatten nicht auf Anthrazyklin, 82% nicht auf ein Therapieregime, das 5-FU beinhaltete, angesprochen.
46% hatten mindestens 2 oder 3 Vortherapien, 85 Patienten (53%) eine adjuvante Vortherapie mit 5-FU. 89% der behandelten Patienten hatten bereits vier oder mehr zytotoxische Substanzen erhalten.
Eine objektive Remissionsrate von 20% wurde in der "intend to treat"- Auswertung (162 Patienten) beobachtet und durch eine unabhängige Kommission bestätigt. Drei beobachtete Komplettremissionen dauerten 106, 109 und 194+ Tage an.
43% der Patienten profitierten durch einen stabilen Krankheitsverlauf. In einer Subgruppenanalyse zeigten 42 der anthrazyklin- und paclitaxeltherapierefraktären Patienten nochmals eine Ansprechrate von 29%.
Die mittlere Remissionsdauer betrug 241 Tage, die mediane Zeit bis zur Progression 93 Tage und das mediane Überleben mit 384 Tagen war für diese intensiv vorbehandelten Patienten überraschend lang.

Nicht nur die antitumoröse Potenz, sondern insbesondere die für dieses Patientenkollektiv wichtige palliative Wirkung in Hinblick auf Schmerzreduktion wurde durch die Studie für Capecitabin belegt. Von 51 Patientinnen, die auf einer analogen Schmerzskala einen Schmerzwert über 20 mm bei Studienbeginn dokumentierten, wurde bei 47% der Schmerz durch die Therapie um mehr als 50 % reduziert.
Die Capecitabintherapie zeigte ein exzellentes Verträglichkeitsprofil. Es war keine Prämedikation oder primäre Prophylaxe notwendig. Therapiebedingte Todesfälle traten nicht auf.

In einer weiteren Phase II Studie wurde Capecitabin in der Second-Line bei Patientinnen nach Anthrazyklinversagen im Vergleich zu Paclitaxel eingesetzt. Patienten mit Capecitabin erzielten zu 36% eine ORR, durch Paclitaxel wurden 21 % Overallresponse erreicht (O'Reilly et al., 1998). Die Studie wurde vorzeitig nach Rekrutierung von 44 Patienten beendet, da viele Patienten klare Präferenzen bezüglich ihrer Medikation hatten, entweder Paclitaxel bevorzugten oder eine orale Substanz. Eine Randomisierung war daher nur schwer durchführbar.
Unter Capecitabin und unter Paclitaxel wurde eine mediane Ansprechdauer von 9,4 Monaten festgestellt. Die mediane Zeit bis zur Tumorprogression lag für Capecitabin bei 3,0 Monaten (92,5 Tage) und für Paclitaxel bei 3,1 Monaten (95 Tage).

Aktuelle Daten einer mit der Rekrutierung abgeschlossenen Phase II Studie, von Reichardt et al., zur Wirksamkeit und Verträglichkeit einer Capecitabintherapie des metastasierten Mammakarzinoms nach einer taxanhaltigen Vortherapie, wurden auf dem San Antonio Breast Cancer Symposium 2000 präsentiert. Bisher waren 97 Patientinnen, die in die Studie eingeschlossen wurden, auswertbar. Medianes Alter 55 Jahre (Range 36-77), mediane Karnofsky-Index 90% (Spannwerte 60-100). 91% der Patientinnen hatten eine anthrazyklinhaltige und 100% eine taxanhaltige Vortherapie (51% Paclitaxel, 45% Docetaxel,4% beide Taxane).
Capecitabin wurde in einer Dosis von 1250mg/m² zweimal täglich, morgens und abends, über 14 Tage gefolgt von einer 7 tägigen Pause, gegeben.
Nach durchschnittlich 4 Zyklen (Range 1-15) sind 77 bzw. 68 Patientinnen auswertbar für Toxizität und Wirksamkeit. Die Toxizität ist mit 40% Hand-Fuß-Syndrom, Übelkeit und Erbrechen 38%, Diarrhoe 21% und Lethargie 18% CTC Grad 1 und 2 mild. Lediglich 14% entwickelten eine Grad 3 Hand-Fuß-Toxizität. 2 Patientinnen (3 %) erreichten eine komplette Remission, 15 (22%) eine partielle und bei 47% kam es zur Krankheitsstabilisation. Insgesamt wurde bei 72% der intensiv vorbehandelten Patientinnen eine Tumorkontrolle erreicht (Reichardt et al, 2000).

Entsprechend einer Phase III Studie (O'Shaughnessy J et al. Superior survival with Capecitabin plus docetaxel combination therapy in anthracycline-pretreated patients with advanced breast cancer: phase III trial results. J Clin Oncol 20 2812-23 (2002) war bei Patientinnen mit metastastasierendem Mammakarzinom zwar eine deutliche Steigerung der Wirkung durch Kombinationstherapie mit Capecitabin und Docetaxel im Vergleich zu Docetaxel zu beobachten, dennoch zeigten auch bei der Kombinationstherapie lediglich 42 % der untersuchten Tumoren ein Ansprechen auf die Behandlung.

Nachteilig am Stand der Technik ist, dass ein prozentual hoher Anteil der Patienten nicht auf die Behandlung mit Capecitabin bzw. Capecitabin in Kombination mit Taxanen anspricht. Da Capecitabin ohne einen vorgeschalteten Nachweis der Thymidinphosphorylase zugelassen ist, werden in der Regel auch solche unempfindlichen bzw. nicht ansprechenden Patienten behandelt, die keinerlei Nutzen von der Therapie haben und unnötigerweise den Nebenwirkungen ausgesetzt werden, ohne dass ein therapeutischer Erfolg eintritt oder zu erwarten wäre.

Molekularbiologisch begründete Therapien sind immer hoch selektive auf die Blockade bestimmter zellulärer Rezeptoren oder Enzyme der Tumorzellen gerichtet. Daraus lässt sich ihre gute Wirksamkeit aber auch im Vergleich zur Chemotherapie das geringe Nebenwirkungsspektrum erklären. Dementsprechend ist für die Durchführung einer antihormonellen Therapie der Nachweis des Östrogen- und Progesteronrezeptors zwingend. Dies gilt in gleicher Weise für die Durchführung einer Herceptintherapie. Hier ist der Nachweis von HER-2 mittels Immunhistochemie (ICH) oder Fluoreszenz in situ Hybridisierung (FISH) notwendig. Die ebenfalls hoch selektiven Medikamente Xeloda, Celebrex und Avastin® haben ihre Zulassung ohne einen vorgeschalteten Nachweis bekommen. Gleichwohl können sie ihre Wirkung nur entfalten, wenn das entsprechende Zielsubstrat im Tumorgewebe vorhanden ist. Deshalb erscheint es notwendig den Einsatz dieser Medikamente auch an den Nachweis der entsprechenden Zielsubstrate zu binden.

Wünschenswert ist deshalb ein Verfahren für die individuelle Feststellung der Wirksamkeit von Capecitabin bzw. seiner im Körper gebildeten Stoffwechselprodukte, ggf. in Kombination mit Docetaxel oder Paclitaxel und/oder Herceptin und/oder COX-2 Hemmern und/oder Angiogenesehemmern, bevor der Wirkstoff bzw. die Wirkstoffkombination Patienten verabreicht wird.

Aufgabe der Erfindung ist es daher, ein Verfahren anzugeben, dass die Identifizierung der Unempfindlichkeit bzw. Nichtansprechbarkeit oder der Empfindlichkeit bzw. Ansprechbarkeit eines humanen soliden Tumors bezüglich einer Behandlung mit Capecitabin ermöglicht, der die einfache Umsetzung der Erfindung ermöglicht.

Diese Aufgabe wird durch ein Verfahren gemäß Anspruch 1 gelöst. Die weiteren Ansprüche sind Vorzugsvarianten der Erfindung.

Die Lösung der Aufgabe besteht deshalb darin, Thymidinphosphorylase (TP) im Tumorgewebe zu bestimmen und als Voraussetzung für den Einsatz des Medikaments Capecitabin nachzuweisen.

Ergänzt wird dieser Befund bzw. Effekt durch den Nachweis unerwartet niedriger Konzentrationen von COX-2 (Cyclooxygenase-2) im Tumorgewebe, insbesondere von unempfindlichen Patientinnen. Dieses Enzym ist die Zielstruktur für die Medikamentengruppe der COX-2 Hemmer, z.B. Celebrex (Celecoxib, 4-[5-(4-methylphenyl)-3-(trifluoromethyl)pyrazol-1-yl]). Es gilt dementsprechend der gleiche Grundsatz, der einen zielgerichteten Einsatz des Medikaments bei der Tumortherapie, insbesondere den kombinierten Einsatz von Capecitabin und COX-2 Hemmern, vorzugsweise von Capecitabin und Celebrex, erst nach dem Nachweis dieses Enzyms vorsieht.

Die Lösung der erfindungsgemäßen Aufgabe besteht auch darin, COX-2 im Tumorgewebe zu bestimmen und für einen verlässlichen Einsatz des Medikaments und ggf. eines weiteren Wirkstoffs nachzuweisen.

Insbesondere die Bestimmung sowohl von TP als auch von COX-2 im Tumorgewebe hat sich vollkommen überraschend als zuverlässiges Instrument für die Vorhersage der Unempfindlichkeit oder Empfindlichkeit humaner Tumoren gegenüber Capecitabin, besonders in Kombination mit COX-2 Hemmern, vorzugsweise mit Celebrex, und/oder in Kombination mit Docetaxel oder Paclitaxel und/oder in Kombination mit Herceptin (Trastuzumab) erwiesen. Entsprechendes gilt für die Bestimmung von VEGF und VEGF-Rezeptoren zur Kombination mit Angiogenesehemmern, z.B. Avastin®.

Die Lösung der erfindungsgemäßen Aufgabe besteht also darin, sowohl TP als auch im Tumorgewebe zu bestimmen und als Voraussetzung für einen zuverlässigen Einsatz des Medikaments und ggf. eines weiteren Wirkstoffs oder mehrerer weiterer Wirkstoffe nachzuweisen.

In der Ausführung der Erfindung wird das Vorhandensein von VEGF und VEGF-Rezeptoren zur Durchführung einer Angiogenesehemmung z.B. mit Avastin® nachgewiesen.

Die Erfindung basiert also im Prinzip darauf, dass in dem Gewebe eines humanen Tumors, insbesondere eines metastasierenden Karzinoms des Dickdarms oder der Brust oder eines anderen soliden malignen, insbesondere metastastasieren, Tumors, die humane Thymidinphosphorylase und die humane Cyclooxygenase 2 und VEGF und VEGF Rezeptoren bestimmt werden.
Abhängig von dem Ergebnis bzw. Ergebnissen dieser Bestimmung erfolgt dann die Verabreichung von Capecitabin, ggf. in Kombination mit Docetaxel und/oder mit Paclitaxel und/oder mit dem humanisierten Antikörper Herceptin und/oder mit COX-2 Hemmern, insbesondere Celebrex und/oder mit einem Angiogenesehemmer, vorzugsweise Avastin®

Für das Verfahren wird vorzugsweise Gewebe oder Gewebematerial, z.B. mittels einer Biopsie, aus dem humanen Tumor isoliert oder bereits isoliertes Gewebematerial verwendet. Für die Isolierung sind prinzipiell alle möglichen Standardverfahren oder andere bekannte Methoden zur Isolierung von Gewebe oder Gewebematerial aus einem soliden Tumor geeignet, bspw. eine Resektion von Tumormaterial aus dem Patienten. Als Gewebe sind vorzugsweise Gewebeschnitte geeignet, aber auch sonstiges Material, das aus Gewebe erhalten wurde, bspw. Gewebehomogenisat, Zellen, Zellbestandteile oder Lösungen, die diese enthalten.

Besonders bevorzugt wird Thymidinphosphorylase und/oder Cyclooxygenase 2 immunhistologisch in dem isolierten Tumorgewebe, vorzugsweise Gewebeschnitten, bestimmt. VEGF und VEGF-Rezeptoren werden ebenso immunhistologisch nachgewiesen.
Geeignet sind dafür insbesondere alle Standardverfahren der Antikörperfärbung, der Immunfärbung und der Immunhistochemie, mittels derer Proteine mit Hilfe von Antikörpern oder anderer spezifischer Liganden in Gewebe sichtbar gemacht werden können, insbesondere durch direkte Methoden (Verwendung markierter Primärantikörper), indirekte Methoden (Verwendung markierter Sekundärantikörper) und/oder die Avidin-Biotin-Methode (Vewendung von biotinylierten Antiköpern und markiertem Streptavidin).
Immunhistologische Methoden zur Bestimmung von TP und/oder COX-2 und/oder VEGF und VEGF-Rezeptoren, insbesondere unter Verwendung von fixiertem Tumorgewebe bzw. -gewebeschnitten, haben dabei insbesondere den Vorteil, dass sie besonders einfach und unmittelbar nach Isolierung des Tumorgewebes durchführbar sind.
Eine andere Möglichkeit für die Umsetzung des Verfahrens besteht darin, dass Zellen aus Tumorflüssigkeit oder -punktaten u.ä., die mittels Zentrifugation oder anderer Verfahren auf einen Träger, insbesondere Objektträger, aufgebracht werden, oder auf einem Träger mittels Zellkulturtechnik aufgewachsen sind, immunhistochemisch für die Bestimmung von TP und/oder COX-2 und/oder VEGF und VEGF-Rezeptoren verwendet werden. Vorzugsweise wird für die Umsetzung des Verfahrens mittels immunhistologischer Methoden die Menge an anti-TP Antikörpern und/oder anti-COX-2 und/oder VEGF und VEGF-Rezeptoren Antikörpern derart niedrig gewählt, dass, abhängig von der verwendeten Markierung und Ausleseapparatur, z.B. einem Fluoreszenz-Mikroskop, eine signifikant erhöhte Menge bzw. Konzentration von TP und/oder COX-2 und/oder VEGF und VEGF-Rezeptoren im Gewebe (gerade noch) erkennbare Signale verursacht, wohingegen eine normale bzw. mit gesundem Gewebe vergleichbare oder niedrigere Konzentrationen von TP und/oder COX-2 und/oder VEGF und VEGF-Rezeptoren nicht mehr erkennbare Signale verursacht, wodurch die Umsetzung des Verfahrens nochmals vereinfacht wird.

Zum Nachweis von TP und/oder COX-2 und/oder VEGF und VEGF-Rezeptoren sind dementsprechend alle möglichen Festphasen-basierten Assays (z.B. ELISAs oder entsprechende Farbstoff-Assays, Westernblots, Southern-Blots, Northern-Blots, Arrays usw.) geeignet.

Nach einem anderen Aspekt wird die Anwesenheit von Thymidinphosphorylase und/oder Cyclooxygenase 2 und/oder VEGF und VEGF-Rezeptoren im Tumorgewebe durch eine Bestimmung ihrer biologischen Funktion nachgewiesen, bspw. durch Zugabe und Vermessung der Umsetzung von Enzymsubstrat durch TP (z.B. von 5'-DFUR) und/oder durch COX-2 (z.B. von Anandamid) im Gewebe oder Gewebematerial. Vorteilhafterweise wird dazu parallel ein Standard mit bekannter Menge an TP und/oder COX-2 vermessen.
Aber auch die Bestimmung der Genexpression von TP und/oder COX-2 und/oder VEGF und VEGF Rezeptoren ist geeignet, um ihre Anwesenheit und Konzentration im Gewebematerial nachzuweisen. Dazu sind insbesondere alle möglichen Standardverfahren zum Nachweis der RNA von TP und/oder COX-2 und/oder VEGF und VEGF-Rezeptoren im Gewebe oder Gewebematerial geeignet. Beispielsweise wird RNA aus dem Gewebe isoliert und mittels RT-PCR in cDNA transkribiert. Die cDNA wird nachfolgend selektiv durch eine PCR mit Primersequenzen aus humanem TP und/oder COX-2 und/oder VEGF und VEGF-Rezeptoren vervielfältigt und die PCR-Produkte gelelektrophoretisch aufgetrennt und bestimmt. Aber auch die Verwendung von Real-Time-PCRs ist geeignet. Günstig ist insbesondere auch die Synthese von cRNA aus der cDNA durch Zweitstrangsynthese, Fragmentierung der Produkte und anschließender Nachweis von TP und/oder COX-2 und/oder VEGF und VEGF-Rezeptoren spezifischen Produkten mittels eines Oligonukleotidarrays, insbesondere Microarrays.

Geeignet sind vorzugsweise alle Verfahren für die Bestimmung von TP und/oder COX-2 und/oder VEGF und VEGF-Rezeptoren, bei denen Tumorgewebe oder Teile davon mit wenigstens einer zumindest teilweise gelösten Substanz in Kontakt gebracht wird, die Affinität zu dem Gen der humanen Thymidinphosphorylase aufweist und/oder zu Varianten und/oder zu Teilen davon und/oder zu seiner mRNA und/oder zu seinen Genprodukten und/oder davon abgeleiteten Spaltprodukten, Polypeptiden oder Peptiden **und/oder** mit mindestens einer Substanz in Kontakt gebracht wird, die Affinität zu dem Gen der humanen Cyclooxygenase 2 aufweist und/oder zu Varianten und/oder zu Teilen davon und/oder zu seiner mRNA und/oder zu seinen Genprodukten und/oder davon abgeleiteten Spaltprodukten, Polypeptiden oder Peptiden **und/oder** mit mindestens einer Substanz in Kontakt gebracht wird, die Affinität zu dem Gen der humanen VEGF und des humanen VEGF-Rezeptors aufweist und/oder zu Varianten und/oder zu Teilen davon und/oder zu seiner mRNA und/oder zu seinen Genprodukten und/oder davon abgeleiteten Spaltprodukten, Polypeptiden oder Peptiden. Die Affinität ist dabei bevorzugt über eine Assoziationskonstante Ka > 1000 M⁻¹ ausgestaltet, wodurch ein besonders spezifischer Nachweis ermöglicht wird. Besonders bevorzugt wird das wenigstens eine Substanz enthaltende Enzymsubstrat von TP und/oder COX-2, Oligonukleotidsequenzen von TP und/oder COX-2 und/oder VEGF und VEGF-Rezeptoren bzw. komplementäre Sequenzen davon, Proteine, Peptide oder davon abgeleitete Strukturen verwendet, insbesondere monoklonale Antikörper und/oder polyklonale Antikörper und/oder Antikörperfragmente, die gegen TP und/oder COX-2 und/oder VEGF und VEGF-Rezeptoren gerichtet sind.

In Weiterbildung ist die wenigstens eine zumindest teilweise gelöste Substanz, vorzugsweise direkt, mit einem Marker, bspw. einem Enzym (z.B. AP oder HRP), verbunden, wodurch ein besonders einfacher Nachweis von TP und/oder COX-2 und/oder VEGF und VEGF-Rezeptoren ermöglicht wird.

Bevorzugt ist der Marker als (Fluoreszenz-)Farbstoff, Kontrastmittel, Radionuklid, Biotin, Peptid, Protein oder Mikropartikel gebildet oder umfasst diese, besonders bevorzugt ist der Marker als markierter Sekundärantikörper oder markiertes Protein A oder markiertes Protein G oder davon abgeleitete Struktur gebildet.

Die Verbindung zwischen der mindestens einen Substanz und dem Marker ist vorzugsweise chemisch, elektrostatisch und/oder über hydrophobe Wechselwirkungen gebildet, besonders geeignet ist eine kovalente Verbindung.

Entsprechend wird ein, humaner Tumor, der unempfindlich bezüglich einer Verabreichung von Capecitabin ist, insbesondere unempfindlich bezüglich einer Verabreichung von Capecitabin in Kombination mit Docetaxel oder Paclitaxel oder Herceptin ist, oder der unempfindlich bezüglich einer Verabreichung von Capecitabin in Kombination mit einem COX-2 Hemmer, z.B. Celebrex, und /oder einem Angiogenesehemmer, z.B. Avastin ist, dadurch identifiziert, dass in dem untersuchten Tumorgewebe Thymidinphosphorylase und/oder Cyclooxygenase 2 und/oder VEGF und VEGF-Rezeptoren im wesentlichen nicht nachweisbar ist und/oder ihre Genexpression oder enzymatische Aktivität signifikant niedriger und/oder nicht signifikant höher (insbesondere TP) ist als in vergleichbarem gesunden Gewebe.
Ein humaner Tumor, der sensitiv bezüglich Capecitabin ist, oder sensitiv bezüglich COX-2 Hemmer oder sensitiv bezüglich einem Angiogenesehemmer, insbesondere sensitiv bezüglich Capecitabin in Kombination mit Docetaxel oder Paclitaxel oder Herceptin ist, oder der sensitiv bezüglich Capecitabin in Kombination mit einem COX-2 Hemmer, vorzugsweise Celecoxib (Celebrex®) oder Etoricoxib (Arcoxia®), oder der sensitiv bezüglich Capecitabin in Kombination mit einem Angiogenesehemmer, vorzugsweise Avastin®, oder der sensitiv bezüglich Capecitabin in Kombination mit einem COX-2 Hemmer, vorzugsweise Celecoxib (Celebrex®) oder Etoricoxib (Arcoxia®) und in Kombination mit einem Angiogenesehemmer, vorzugsweise Avastin®, ist, wird dadurch identifiziert, dass Thymidinphosphorylase und/oder Cyclooxygenase 2 und/oder VEGF und VEGF-Rezeptoren in seinem Tumorgewebe nachweisbar sind und/oder ihre Genexpression oder enzymatische Aktivität signifikant höher und/oder vergleichbar (insbesondere COX-2) zu entsprechendem gesundem Gewebe ist.

Bspw. werde folgende Ergebnisse bei der Bestimmung von TP und COX-2 im Tumorgewebe erhalten:
a)TP und/oder COX-2 ist nicht nachweisbar und/oder ihre Konzentration ist signifikant niedriger als in entsprechendem gesunden Gewebe:
   Der Tumor wird als unempfindlich bezüglich einer Behandlung des Patienten mit Catecitabin, ggf. in Kombination mit einem weiteren Medikament identifiziert.
b)TP und/oder COX-2 ist nachweisbar und/oder ihre Konzentration ist signifikant höher als in entsprechendem gesunden Gewebe:
   Der Tumor wird als empfindlich gegenüber einer Behandlung mit Capecitabin, ggf. in Kombination mit einem weiteren Medikament, insbesondere in Kombination mit einem COX-2 Hemmer, vorzugsweise Celebrex, identifiziert.
c)TP ist nachweisbar und/oder ihre Konzentration ist signifikant höher als in entsprechendem gesunden Gewebe **und/oder** COX-2 ist nicht nachweisbar und/oder ihre Konzentration ist signifikant niedriger als in entsprechendem gesunden Gewebe. Der Tumor wird als empfindlich gegenüber einer Behandlung mit Capecitabin, ggf. in Kombination mit einem weiteren Medikament, insbesondere in Kombination mit Docetaxel oder Paclitaxel oder Herceptin identifiziert.
d)TP ist nachweisbar und/oder ihre Konzentration ist signifikant höher als in entsprechendem gesunden Gewebe **und/oder** COX-2 ist nachweisbar und/oder ihre Konzentration ist vergleichbar mit entsprechendem gesunden Gewebe:
   Der Tumor wird als empfindlich gegenüber einer Behandlung mit Capecitabin, ggf. in Kombination mit einem weiteren Medikament, insbesondere in Kombination mit einem COX-2 Hemmer, vorzugsweise Celebrex, identifiziert.
e)TP ist nicht nachweisbar oder ihre Konzentration ist signifikant niedriger als in entsprechendem gesunden Gewebe und/oder ihre Konzentration ist vergleichbar mit entsprechendem gesunden Gewebe **und/oder** COX-2 ist nachweisbar und/oder ihre Konzentration ist vergleichbar mit entsprechendem gesunden Gewebe und/oder ihre Konzentration ist signifikant höher als in entsprechendem gesunden Gewebe:
   Der Tumor wird als unempfindlich gegenüber einer Behandlung mit Capecitabin, ggf. in Kombination mit einem weiteren Medikament, und als empfindlich gegenüber einer Behandlung mit einem COX-2 Hemmer, vorzugsweise Celebrex, identifiziert.

Ein anderer Aspekt, der sich aus den Ergebnissen der Arbeiten ergibt, betrifft die Verwendung von Capecitabin und COX-2 Hemmern, vorzugsweise Capecitabin und Celebrex und Angiogenesehemmern, vorzugsweise Avastin® für die Herstellung eines Medikaments gegen einen humanen Tumor, insbesondere gegen ein metastasierendes Karzinom des Dickdarms oder der Brust oder einen anderen soliden, insbesondere metastasierenden, Tumor. Das Medikament ist vorzugsweise für die Therapie von Patienten geeignet, wenn zuvor in dem Gewebe des zu behandelnden Tumors TP und/oder COX-2 und/oder VEGF und VEGF-Rezeptoren bestimmt und festgestellt wurden.

Die Offenbarung ermöglicht erstmals eine Vorhersage des Behandlungserfolgs für Patienten mit soliden Tumoren, insbesondere mit einem metastasierenden Karzinom des Dickdarms oder der Brust oder einem anderen soliden, insbesondere metastasierenden, Tumor, gegenüber einer Behandlung mit Capecitabin, insbesondere in Kombination mit Docetaxel oder Paclitaxel oder Herceptin oder einem COX-2 Hemmer, vorzugsweise Celebrex und/ oder in Kombination mit Angiogenesehemmern, vorzugsweise Avastin®. Der Einsatz dieser Medikamente wird an den Nachweis der entsprechenden Zielsubstrate gebunden, um die Effektivität dieser Therapien deutlich zu erhöhen.
Die Möglichkeiten für das Erstellen eines Onkobiogramms werden mittels der Anwendung von vielen verschiedenen Targets erheblich erweitert. Dabei werden vor allem Tumoreigenschaften, die das Ziel für neue Therapien sind, oder Marker, die genaue Aussagen über die Bösartigkeit des Tumorgewebes zulassen bestimmt. Damit eröffnen sich Behandlungschancen vor allem für diejenigen Patientinnen, bei denen alle etablierten Therapien fehlgeschlagen sind.
Patienten die unempfindlich gegenüber einer solchen Behandlung sind, bleiben durch die erfindungsgemäße Identifizierung die Nebenwirkungen dieser medikamentösen Behandlung erspart und es muss auch nicht erst der fehlgeschlagene Behandlungsversuch abgewartet werden, bevor weitere Maßnahmen zur ihrer Lebenserhaltung getroffen werden können.

Weitere vorteilhafte Merkmale werden auch aus den nachfolgenden Beispielen und Erläuterungen ersichtlich.

Vollkommen überraschend wurde in den Untersuchungen, die zu der Erfindung geführt haben, festgestellt, dass der immunhistologische Nachweis von Thymidinphosphorylase im Tumorgewebe kausal mit einer ausgeprägten Verlängerung der TTP (time to progression) einhergeht. Patientinnen ohne Expression des Enzyms haben dagegen keinen Nutzen von der Therapie.

In einer innerhalb der erfindungsgemäßen Arbeiten durchgeführten prospektiven Phase II-Studie wurde die Wirkung der Medikamentenkombination Capecitabin/Trastuzumab beim metastasierten Mammakarzinom überprüft. Bei beiden Medikamenten handelt es sich um hoch zielgerichtete Therapeutika. Capecitabin benötigt für seine Wirksamkeit die TP und Trastuzumab HER-2 (Human Epidermal growth Factor Receptor 2). Einschlußkriterien waren Krankheitsprogression nach Abschluß anthrazyklin- und taxanhaltiger Chemotherapien und der Nachweis von HER-2 aber nicht von TP. Nach einer Beobachtungszeit von 34 Monaten hatten 4 (17%) von 27 Patientinnen eine komplette Remission der Erkrankung, 8 (35%) eine partielle Remission, 9 (39%) eine stable disease. Bei 2 (9%) schritt die Erkrankung unvermindert fort. (Schaller G et al.: Phase II Study of Capecitabine Plus Trastuzumab in Human Epidermal Growth Factor Receptor 2-Overexpressing Metastatic Breast Cancer Pretreated With Anthracyclines or Taxanes. J Clin Oncol. (2007) Jun 18).

Auffällig bei dieser Studie war, dass sich bei vier Patientinnen eine ungewöhnlich langanhaltende und stabile komplette Remission eingestellt hatte, was bei der extrem ungünstigen Ausgangssituation äußerst überraschend war.

Es wurde die Hypothese formuliert, dass das gute Ansprechen im Zusammenhang mit der TP-Expression im Tumor stehen könnte. Folglich wurde bei den Studienpatientinnen retrospektiv TP intratumoral, peritumoral und als Gesamtfraktion zusmmen mit COX-2, VEGF und K18 (Keratin 18) immunhistologisch bestimmt. Diese Parameter wurden dann zusammen mit 30 weiteren biologischen, therapeutischen und anmnestischen Daten einer Analyse mit Hilfe der mathematischen Logik (Klaus Truemper: Design of Logic-based Intelligent Systems, published by John Wiley & Sons 2004) unterzogen. Aus organisatorischen Gründen standen nur 14 Proben für die Analyse zur Verfügung. Das wesentliche Ergebnis war, dass die Faktorenkombination starke Expressionswerte für TP und niedrige für COX-2 entscheidend für eine lange TTP waren.

Dieses Ergebnis wird anhand von zwei Patientenverläufen näher ausgeführt.

**Patientin 1** ist jetzt 48 Jahre alt. 1995 wurde die operative Primärtherapie in Form einer Ablatio mammae rechts durchgeführt. Histologisch handelte es sich um ein pT2 (ø 2,8 cm), pN1 (2/18 Lk befallen), pMx duktales Mammakarzinom, G2. An die Operation schloß sich eine adjuvante Chemotherapie mit CMF an, 1997 trat das erste Brustwandrezidiv auf, das operative entfernt wurde. Anschließen wurde lokal bestrahlt. 1999 wurde ein weiteres Brustwandrezidiv operativ entfernt. Im gleichen Jahr traten erstmals Knochenmetastasen (linke Hüfte, Os occipitale inkl. Weichteile) und Hautmetastasen (rechts supraklavikulär und diffus rechte Thoraxwand) auf. Es erfolgte erstmals die Gabe von Trastuzumab (Herceptin) in Kombination mit verschiedenen antihormonellen Therapien (Tamoxifen, Aromatasehemmer, GnRH-Analoga, Ovarektomie). In 12/03 wurde erstmal Capecitabin (Xeloda) in Kombination mit Trastuzumab eingesetzt, das bis jetzt andauernd eingenommen wird. Die Knochenmetastasen wurden bestrahlt. Unter dieser Behandlung konnte eine komplette Remission erzielt werden, die bislang nahezu drei Jahre andauert. Am 9. 4. 2006 wurde ein Onkobiogramm durchgeführt. Dabei zeigten sich eine starke Expression der Thymidinphosphorylase sowohl im Tumor (IRS 9) als auch peritumoral (IRS 9) und niedrige Werte für die COX-2 (IRS 2). Der Patientin geht es sehr gut. Sie arbeitet in ihrem Beruf als Friseurin und ist gerade von einer drei-wöchigen Thailandreise zurückgekehrt.

**Patientin 2** ist jetzt 69 Jahre alt. 1999 wurde die Primärtherapie in Form einer Teilmastektomie mit axillärer Dissektion durchgeführt. Es handelte sich um ein multifokales duktales Mammakarzinom, pT2, pN 1 biii (7/15), G3, DCIS G2 (30%). Es schloß sich eine adjuvante Chemotherapie mit 4xEC, eine Bestrahlung der Restbrust gefolgt von 4xTaxöl an. Der Tumor exprimierte den Östrogenrezeptor. Deshalb wurde von 10/99 bis 9/01 mit Tamoxifen behandelt. Im August 2001 wurde eine solitäre Lebermetatsase ø 3cm sonografisch und kernspintomografisch nachgewiesen. Die zu diesem Zeitpunkt durchgeführte HER-2-Bestimmung ergab eine Überexpression von 3+. Es erfolgte der Einschluß in die Capecitabin/Trastuzumab-Studie. Darunter kam es zu einer kompletten Remission, die bislang andauert. Die retrospktive Bestimmung von TP und COX-2 ergab eine ausgeprägte Expression für TP im Tumor (IRS 6) als auch besonders peritumoral (IRS 9) und eine schwache Expression (IRS 2) von COX-2. Der Patientin geht es gut. Sie hat gerade mit ihrem Ehemann ein neues Haus gebaut.
Figur 1 zeigt die Umwandlung und Wirksamkeit von Capecitabin im Körper

## Patentansprüche

1. Verfahren zur Identifizierung eines humanen Tumors, der unempfindlich oder empfindlich bezüglich Capecitabin, ggf. in Kombination mit einem COX-2 Hemmer, vorzugsweise Celecoxib oder einem Angiogenesehemmer, vorzugsweise Bevacizumab, ist, insbesondere eines metastasierenden Karzinoms des Dickdarms oder der Brust oder eines anderen soliden Tumors, wobei in dem isolierten Tumorgewebe Thymidinphosphorylase und Cyclooxygenase 2 und VEGF und VEGF-Rezeptoren bestimmt werden, **dadurch gekennzeichnet, dass** Abhängig von dem Ergebnis bzw. Ergebnissen dieser Bestimmung die Verabreichung von Capecitabin, ggf. in Kombination mit Docetaxel und/oder mit Paclitaxel und/oder mit dem humanisterten Antikörper Herceptin und/oder mit COX-2 Hemmern, insbesondere Celecoxib und/oder mit einem Angiogenesehemmer, vorzugsweise Bevacizumab erfolgt.

2. Verfahren nach Anspruch 1, **dadurch gekennzeichnet, dass** die Bestimmung der - Thymidinphosphorylase, der Cyclooxygenase 2 und der VEGF und VEGF-Rezeptoren immunhistologisch erfolgt.

3. Verfahren nach einem der vorangehenden Ansprüche, **dadurch gekennzeichnet, dass** die Genexpression oder die biologische Funktion von Thymidinphosphorylase, der Cyclooxygenase 2 und der VEGF und VEGF-Rezeptoren bestimmt wird.

4. Verfahren nach einem der vorangehenden Ansprüche, **dadurch gekennzeichnet, dass** das Tumorgewebe oder Teile davon mit wenigstens einer zumindest teilweise gelösten Substanzen in Kontakt gebracht wird, die Affinität zu dem Gen der humanen Thymidinphosphorylase aufweist oder zu seinen Genprodukten, Polypeptiden oder Peptiden, und/oder mit mindestens einer Substanz, die Affinität zu dem Gen der humanen Cyclooxygenase 2 aufweist oder zu seinen Genprodukten , Polypeptiden oder Peptiden, und/oder mit mindestens einer Substanz die Affinität zu dem Gen der humanen VEGF und des humanen VEGF-Rezeptors aufweist oder zu seinen Genprodukten, Polypeptiden oder Peptiden.

5. Verfahren nach Anspruch 4, **dadurch gekennzeichnet, dass** die wenigstens eine Substanz als Enzymsubstrat, Oligonukleotid, Protein, Peptid oder davon abgeleitete Struktur gebildet ist.

6. Verfahren nach einem der Ansprüche 4-5, **dadurch gekennzeichnet, dass** die wenigstens eine Substanz als monoklonale Antikörper und/oder polyklonale Antikörper und/oder Antikörperfragmente gebildet ist.

7. Verfahren nach einem der Ansprüche 4-5, **dadurch gekennzeichnet, dass** ein Marker chemisch, elektrostatisch und/oder über hydrophobe Wechselwirkungen mit mindestens einer der wenigstens zwei Substanzen verbunden ist.

8. Verfahren nach einem der nprüche 1-7 zur Identifizierung eines humanen Tumors, der unempfindlich bezüglich Capecitabin, allein oder Capecitabin in Kombination mit Dicetaxel, Paclitaxel oder Trastuzumab oder einem COX-2 Hemmer, vorzugsweise Celecoxib, und/oder einem Angiogenesehemmer, vorzugsweise Bevacizumab ist, **dadurch gekennzeichnet, dass** in dem untersuchten Tumorgewebe Thymidinphosphorylase und Cyclooxygenase 2 und/oder VEGF und VEGF Rezeptoren nicht nachweisbar sind und/oder ihre Genexpression oder enzymatische Aktivität nicht signifikant höher ist als in vergleichbaren gesunden Gewebe ist.

9. Verfahren nach einem der Ansprüche 1-7 zur Identifizierung eines humanen Tumors, der sensitiv bezüglich Capecitabin allein oder Capecitabin in Kombination mit Docetaxel, Paclitaxel oder Trastuzumab oder einem COX-2 Hemmer, vorzugsweise Celecoxib, und/oder einem Angiogenesehemmer, vorzugsweise Bevacizumap ist, **dadurch gekennzeichnet, dass** Thymidinphosphorylase allein oder Thymidinphosphorylase in Kombination mit Cyclooxygenase 2 und/oder VEGF und VEGF Rezeptoren im Tumorgewebe nachweisbar sind und/oder ihre Genexpression oder enzymatische Aktivität signifikant höher im Vergleich zu entsprechendem gesunden Gewebe ist.

## Claims

1. Method for identification of a human tumor which is insusceptible or susceptible to Capecitabine, possibly in combination with a COX-2 inhibitor, preferably Celecoxib or an angiogenesis inhibitor, preferably Bevacizumab, particularly of a metastasizing colon or breast carcinoma or of any other solid tumor, wherein in the isolated tumor tissue thymidine phosphorylase and cyclooxygenase 2 and VEGF and VEGF receptors are determined, **characterized in that** the application of Capecitabine, possibly in combination with Docetaxel and/or Paclitaxel and/or the humanized antibody Herceptin and/or COX-2 inhibitors, particularly Celecoxib and/or angiogenesis inhibitor, preferably Bevacizumab, is performed depending on the result or the results of this determination.

2. Method according to claim 1, **characterized in that** the determination of the
- thymidine phosphorylase, the cyclooxygenase 2 and the VEGF and VEGF receptors is performed immunhistologically.

3. Method according to any of the previous claims, **characterized in that** the gene expression or the biological function of thymidine phosphorylase, cyclooxygenase 2 and the VEGF and VEGF receptors is determined.

4. Method according to any of the previous claims, **characterized in that** the tumour tissue or parts of it are contacted with at least one at least partially dissolved substance which has affinity to the gene of the human thymidine phosphorylase or to its gene products, polypeptides or peptides, and/or with at least one substance which has affinity to the gene of the human cyclooxygenase 2 or to its gene products, polypeptides or peptides, and/or with at least one substance which has affinity to the gene of the human VEGF and the human VEGF receptor or to its gene products, polypeptides or peptides.

5. Method according to claim 4, **characterized in that** at least one substance is formed as an enzyme substrate, oligonucleotide, protein, peptide or structure derived thereof.

6. Method according to claim 4 or 5, **characterized in that** the at least one substance is formed as monoclonal antibodies and/or polyclonal antibodies and/or antibody fragments.

7. Method according to claim 4 or 5, **characterized in that** a marker is connected chemically, electrostatically and/or by hydrophobic interactions with at least one of the at least two substances.

8. Method according to any of claims 1 - 7 for the identification of a human tumour which is insusceptible to Capecitabine alone or Capecitabine in combination with Docetaxel, Paclitaxel or Trastuzumab or a COX-2 inhibitor, preferably Celecoxib, and/or an angiogenesis inhibitor, preferably Bevacizumab, **characterized in that** thymidine phosphorylase and cyclooxygenase 2 and/or VEGF and VEGF receptors are not determinable in the investigated tumour tissue and/or their gene expression or enzymatic activity is not significantly higher than in comparable healthy tissue.

9. Method according to any of claims 1 - 7 for identification of a human tumour which is sensitive to Capecitabine alone or Capecitabine in combination with Docetaxel, Paclitaxel or Trastuzumab or a COX-2 inhibitor, preferably Celecoxib, and/or an angiogenesis inhibitor, preferably Bevacizumab, **characterized in that** thymidine phosphorylase alone or thymidine phosphorylase in combination with cyclooxygenase 2 and/or VEGF and VEGF receptors is determinable in the tumour tissue and/or their gene expression or enzymatic activity is not significantly higher as compared to corresponding healthy tissue.

## Revendications

1. Procédé d'identification d'une tumeur humaine insensible ou sensible à la capécitabine, éventuellement combinée à un inhibiteur de la COX-2, de préférence à du célécoxibe ou à un inhibiteur de l'angiogénèse, de préférence à du bevacizumab, notamment d'un carcinome métastasant du gros intestin ou du sein ou d'une autre tumeur solide, consistant à mesurer dans le tissu tumoral isolé la thymidinephosphorylase et la cyclo-oxygénase 2 et le VEGF et les récepteurs du VEGF et **caractérisé en ce qu'**en fonction du ou des résultat(s) de cette mesure, il y aura administration de capécitabine, le cas échéant combinée au docétaxel et/ou au paclitaxel et/ou à l'anticorps humanisé Herceptine et/ou aux inhibiteurs de la COX-2, notamment au célécoxibe et/ou à un inhibiteur de l'angiogénèse, de préférence au bevacizumab.

2. Procédé selon la revendication 1, **caractérisé en ce que** la mesure de la
- thymidinephosphorylase, de la cyclo-oxygénase 2 et du VEGF et des récepteur du VEGF se fait par examen immunohistologique.

3. Procédé selon l'une quelconque des revendications précédentes, **caractérisé par** la mesure de l'expression génique ou de la fonction biologique de la thymidinephosphorylase, de la cyclo-oxygénase 2 et du VEGF et des récepteurs du VEGF.

4. Procédé selon l'une quelconque des revendications précédentes, **caractérisé en ce que** le tissu tumoral ou des parties de celui-ci sont mises en contact avec au moins une substance au moins en partie dissoute présentant une affinité pour le gène de la thymidinephosphorylase humaine ou ses produits géniques, des polypeptides ou des peptides, et/ou avec au moins une substance présentant une affinité pour le gène de la cyclo-oxygénase 2 humaine ou ses produits géniques, des polypeptides ou des peptides et/ou avec au moins une substance présentant une affinité pour le gène du VEGF humain et du récepteur du VEGF humain ou ses produits géniques, des polypeptides ou peptides.

5. Procédé selon la revendication 4, **caractérisé en ce qu'**au moins une substance soit formée comme substrat enzymatique, oligonucléotide, protéine, peptide ou comme structure dérivée de ceux-ci.

6. Procédé selon l'une quelconque des revendications 4 ou 5, **caractérisé en ce qu'**au moins une substance soit formée comme anticorps monoclonaux et/ou anticorps polyclonaux et/ou fragments d'anticorps.

7. Procédé selon l'une quelconque des revendications 4 ou 5, caractérisé en ceci qu'un marqueur soit lié chimiquement, électrostatiquement et/ou par interactions hydrophobes avec au moins l'une des substances au nombre d'au moins deux.

8. Procédé selon l'une quelconque des revendications 1 à 7 d'identification d'une tumeur humaine insensible à la capécitabine seule ou à la capécitabine combinée au docétaxel, au paclitaxel ou au trastuzumab ou à un inhibiteur de la COX-2, de préférence au célécoxib, et/ou à un inhibiteur de l'angiogénèse, de préférence au bevacizumab, **caractérisé en ce que** ne puissent être mis en évidence dans le tissu tumoral examiné ni thymidinephosphorylase et cyclo-oxygénase 2 et/ou VEGF et récepteurs du VEGF et/ou que leur expression génique ou leur activité enzymatique ne soient pas significativement supérieures à celles d'un tissu sain comparable.

9. Procédé selon l'une quelconque des revendications 1 à 7 d'identification d'une tumeur humaine sensible à la capécitabine seule ou à la capécitabine combinée au docétaxel, au paclitaxel ou au trastuzumab ou à un inhibiteur de la COX-2, de préférence au célécoxib, et/ou à un inhibiteur de l'angiogénèse, de préférence au bevacizumab, **caractérisé en ce que** ne puissent être mis en évidence dans le tissu tumoral examiné ni thymidinephosphorylase seul ni thymidinephosphorylase combinée à la cyclo-oxygénase 2 et/ou au VEGF et récepteurs du VEGF et/ou que leur expression génique ou leur activité enzymatique soit significativement supérieure à celles d'un tissu sain comparable.
